# EUROPEAN PATENT SPECIFICATION

(11) **EP 0 557 305 B1**
(45) Date of publication and mention of the grant of the patent: **11.06.1997**
(21) Application number: 91918183.4
(22) Date of filing: 24.09.1991
(51) Int. Cl.: C07D 333/20, C07D 409/12, A61K 31/38

(54) **(2-THIENYL) ALKYLAMINE DERIVATIVES HAVING NEUROPROTECTIVE PROPERTIES**
(2-THIENYL)ALKYLAMINDERIVATE MIT NERVENSCHÜTZENDEN EIGENSCHAFTEN
DERIVES DE (2-THIENYL) ALKYLAMINE POSSEDANT DES PROPRIETES NEUROPROTECTRICES

(30) Priority: 26.09.1990 US 589963; 26.09.1990 US 589879
(43) Date of publication of application: 01.09.1993
(73) Proprietor: Astra Aktiebolag, 151 85 Södertälje (SE)
(72) Inventor: GRIFFITH, Ronald, Conrad, Pittsford, NY 14534 (US); SCHMIESING, Richard, Jon, Pittsford, NY 14450 (US); MURRAY, Robert, John, Rochester, NY 14618 (US)
(74) Representative: England, Christopher David
(86) International application number: US9106951
(87) International publication number: WO9205169

(56) References cited:
- EP-A- 0 297 782
- EP-A- 0 298 703
- EP-A- 0 322 582
- EP-A- 0 346 791
- Chemical Abstracts, vol. 114, no. 9, 4 March 1991, Columbus, Ohio, US, S. FIELDING et al.
- Arzneimittel Forschung, vol. 31, no. 10, 1981, Aulendorf, DE, R.I. Mrongovius et al.

## Description

This invention relates to novel (2-thienyl)alkylaminoacetamide derivatives, processes for their preparation, pharmaceutical formulations containing them and their neuroprotective properties.

### Background

Compounds which possess N-methyl-(D)-aspartate (NMDA) blocking properties are useful in the treatment and/or prevention of neurodegeneration in pathological conditions such as stroke, cerebral ischaemia, cerebral palsy, hypoglycaemia, epilepsy, Alzheimer's disease, Huntington's chorea, Olivo-ponto-cerebellar atrophy, perinatal asphyxia and anoxia.

European Patent Application publication number 0322582 discloses, among many other compounds, ortho-substituted alpha-(2-thienyl)benzeneethanamines with anticonvulsant properties.

Mrongovius et al (Chem Abs. 96(5), 28273x) discloses alpha-(2-thienyl)bezeneethanamines as potential non-stimulant anorectics.

Takahashi et al (Chem. Abs. 99(13), 105648q) disclose the synthesis and analgesic activity of alpha-(2-thienyl)benzeneethanamines.

European Patent Application No. 346791 (Searle) discloses 1,2-diaryethylamine derivatives having neuroprotective properties.

Japanese Kokai 58172350 (abstracted in Chem. Abs 100(13), 102914b) discloses alpha-(2-thienyl)benzeneethanamines as analgesics.

### Detailed Description

According to the invention, we provide compounds of formula I, 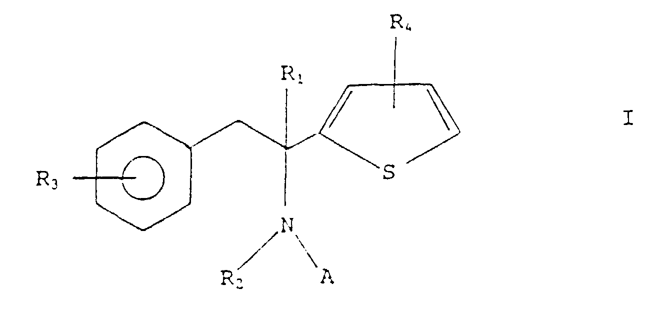 in which:
R₁ represents hydrogen or C₁₋₆ alkyl,
R₂ represents hydrogen, C₁₋₆ alkyl, C₃₋₆ alkenyl, C₃₋₆ alkynyl or C₃₋₆ cycloalkyl,
R₃ represents one or more radicals selected from hydrogen, hydroxy, C₁₋₆ alkyl, C₁₋₆ alkoxy, halogen, trifluoromethyl, amino, C₁₋₆ alkylamino or di(C₁₋₆ alkyl)amino,
R₄ represents one or more radicals selected from hydrogen or C₁₋₆ alkyl,
A represents COCH₂NH₂,
pharmaceutically acceptable acid addition salts thereof.

Pharmaceutically acceptable acid addition salts of the compounds of formula I include salts of mineral acids, for example, hydrohalic acids, e.g. hydrochloric or hydrobromic; or organic acids, for example, formic, acetic or lactic acids. The acid may be polybasic, for example, sulphuric, fumaric, maleic or citric acid.

Certain compounds of formula I may exist in different stereoisomeric forms, including optical enantiomeric forms. All are included within the scope of the invention.

According to the invention, there is also provided the use of a compound of formula I, and pharmaceutically acceptable acid addition salts thereof, in the manufacture of a medicament for use in the prevention and/or treatment of neurological disorders.

Specific neurological disorders that may be mentioned include stroke, cerebral ischaemia, cerebral palsy, hypoglycaemia, epilepsy, Alzheimer's disease, Huntington's chorea, olivo-ponto-cerebellar atrophy, perinatal asphyxia, Parkinsons, anoxia and neuronal damage associated with substance abuse, for example, narcotics or cocaine.

According to another aspect of the invention, there is provided a process for the preparation of the compounds of formula I or pharmaceutically acceptable acid addition salts thereof, which comprises
(a) producing a compound of formula I by reacting the corresponding compound of formula I in which A represents hydrogen with a compound of the formula II, 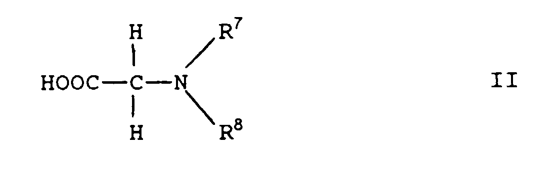 or a carboxyl activated derivative thereof, in which both R₇ and R₈ represent a nitrogen protecting group or one of R₇ and R₈ represents a nitrogen protecting group and the other represents hydrogen; or
(b) producing a compound of formula I by reacting the corresponding compound of formula I in which A represents COCH₂X and X represents a suitable leaving group with ammonia; or (c) producing a compound of the formula I in which one of R₃ represents an NH₂ group by reduction of the corresponding compound of formula I in which one of R₃ represents a NO₂ group; or
(d) producing a compound of formula I containing one or more amino or hydroxy groups by removing a protecting group from a compound of formula I in which one or more of the amino or hydroxyl groups is protected;
and where desired or necessary converting the resulting compound of formula I into a pharmaceutically acceptable acid addition salt thereof or vice versa.

The reaction of process a) may be carried out in conditions similar to those used for the synthesis of peptide bonds in protein chemistry, e.g. by carrying out the reaction in the presence of N,N'-carbonyldiimidazole in a polar aprotic solvent or using a hindered base, e.g. triethylamine and an alkyl chloroformate. One particularly suitable protecting group is benzyloxycarbonyl, which may readily be removed by hydrogenolysis or hydrogen bromide in acetic acid. Other groups that may be mentioned include t-butyloxycarbonyl, (Boc), which is removed by standing the peptide in cold trifluoroacetic acid; Fmoc, which may be removed by treatment with dilute piperidine (20% in DMF); (4-methoxybenzyl)oxycarbonyl and 2-nitrophenylsulphenyl. Further protecting groups and methods for their removal are decribed in T W Greene, Protective Groups in Organic Synthesis, Wiley-Interscience, 1981.

In the reaction of process (b), suitable leaving groups represented by X include halogen, preferably chlorine or bromine, alkyl- or arylsulfonate radicals; for example, methyl sulfonate or p-toluene sulfonate. The amination reaction may be carried out in the absence of a solvent or in the presence of suitable solvent, for example, ethanol or methylene chloride. The reactions may be carried out at a temperature of, for example, from -80°-100°C. In the case where the amine reactant is volatile, for example, ammonia, the reaction may be carried out in a sealed reaction vessel at elevated pressure.

The reduction of process (c) may be carried out by a number of methods for reducing nitro groups as described in Advanced Organic Chemistry (Wiley-Interscience, Ed. J March, 3rd Edition). A particularly preferred method is that of catalytic hydrogenation. Hydrogenation may be carried out in the presence of a catalyst, for example, platinum or palladium, in an appropriate solvent, for example, acetic acid, ethyl acetate or an alcohol, for example ethanol, and at a temperature of, for example, from 0°-100°C.

In the reaction of process (d), removal of the protecting group depends on the nature of the protecting group and includes acidic or basic cleavage or hydrogenolysis. Suitable amine protecting groups are, for example, ethoxycarbonyl, benzyloxycarbonyl, t-butyloxycarbonyl or C₁₋₃ alkanoyl.

Acid addition salts of compounds of formula I may be converted to the corresponding free-base by the action of a stronger base. The acid addition salts of the compound of formula I may be prepared by reaction of the free base with an appropriate acid.

The starting materials for the products of reactions (a) and (c) are either well-known or may be prepared from compounds known per se by conventional methods or by modifications thereof as described in the examples. The following references provide methods for their preparation:

European Patent Application, publication number 0322582; Mrongovius et al (Chem Abs. 96(5), 28273x); Takahashi et al (Chem. Abs. 99(13), 105648q); and Japanese Kokai 58172350(abstracted in Chem. Abs 100(13), 102914b).

The starting materials for the products of reaction (b) may be prepared from the compounds of formula I in which A is hydrogen by conventional acylation techniques, for example, by reaction with an activated carboxylic acid derivative, for example, acetyl chloride, or the derivative may contain a leaving group alpha to the carbonyl group, for example, chloracetyl chloride or 2-bromopropionyl chloride, in the presence of an acid acceptor, for example, triethylamine or pyridine.

In the compound of formula I,
alkyl groups which R₁, R₂, R₃ and R₄ may represent include methyl, ethyl, propyl, isopropyl, n-butyl, iso-butyl and s-butyl;
alkenyl groups which R₂ may represent include 2-propenyl, 2-butenyl and 2-methyl-2-propenyl.
alkynyl groups which R₂ may represent include 2-propynyl and 2-butynyl.
cycloalkyl groups which R₂ may represent include cyclopropyl, cyclobutyl, cyclopentyl and cyclohexyl;
alkoxy groups which R₃ may represent include methoxy, ethoxy and propoxy;
halogen groups which R₃ may represent include fluorine, chlorine, bromine or iodine;

We prefer compounds of formula I or a pharmaceutically acceptable acid addition salt thereof, in which;
R₁ represents hydrogen, methyl, ethyl or isopropyl, preferably hydrogen or methyl;
R₂ represents hydrogen, methyl, ethyl or isopropyl, preferably hydrogen or methyl;
R₃ represents hydrogen, hydroxy, amino or chloro, preferably hydrogen or amino;
R₄ represents hydrogen or methyl;

We especially prefer compounds in which R₃ is hydrogen.

We especially prefer compounds in which R₄ is methyl.

Certain compounds of formula I and their pharmaceutically acceptable acid addition salts are useful because they possess pharmacological activity in animals.

The compounds have useful neuroprotective properties. In particular, they possess NMDA blocking properties. Neurodegeneration is known to be caused or accelerated by certain excitatory amino acids found naturally in the central nervous system (CNS). Glutamate is an endogenous amino acid which has been characterized as a fast excitatory transmitter in the mammalian brain. Glutamate is also known as a powerful neurotoxin capable of killing CNS neurons under certain pathologic conditions which accompany stroke and cardiac arrest. It has been shown that the sensitivity of central neurons to hypoxia and ischemia can be reduced by the specific antagonism of post synaptic glutamate receptors. Glutamate is characterized as a broad spectrum agonist having activity at four neuronal excitatory amino acid receptor sites. These receptor sites are named after the amino acids which selectively excite them: kainate (KA), N-methyl-D-aspartate (NMDA), quisqualate (QUIS) and 2-amino-4-phosphonobutyrate (APB). Glutamate is believed to be a mixed agonist capable of binding to and exciting all three receptor types. Thus, agents which selectively block or antagonise the action of glutamate at these receptors can prevent neurotoxic injury associated with anoxia, hypoxia or ischemia. In particular, compounds which bind to the NMDA receptor site and selectively block the action of glutamate are useful in the prevention and treatment of neurodegenerative diseases.

In addition, certain compounds of formula I demonstrate anticonvulsant activity by their ability to inhibit maximal electroshock (MES) induced seizures in mice; certain compounds inhibit the onset of convulsions and death induced by administration of NMDA to mice; and certain compounds demonstrate antihypoxia activity by their ability to increase the survival time of mice in an oxygen depleted environment.

Antiepileptic activity may be measured by assessing a compound's ability to prevent the hind limb tonic extension component of the seizure in groups of mice induced by maximal electroshock (MES) after oral or intraperitoneal administration, according to the procedures of the Epilepsy Branch, NINCDS as published by R. J. Porter, et al., Cleve. Clin. Quarterly 1984, 51, 293, and compared to the standard agents dilantin and phenobarbital. Activities (ED₅₀'s) in the range of 10-400 m/k after oral administration in this assay system were obtained.

Certain compounds of this invention may possess useful antihypoxia activity. This activity may be conveniently measured in mice. Groups of mice are tested at various times after the intraperitoneal administration of graded doses of the test compound. The animals' survival time in a temperature-controlled hypoxic environment (96% nitrogen and 4% oxygen) is recorded. A statistical comparison is made between coincident vehicle treated animals and the experimental group. The dose-response and minimum active dose (MAD) for compounds are obtained. Other modes of administration can also be used.

NMDA activity may be measured in several ways:
a) NMDA blocking activity is measured by assessing a compound's ability to protect mice from convulsions induced by intravenous administration of 150 m/k of NMDA according to the procedures of Czuczwar et al., (Neurotransmitters, Seizures and Epilepsy III, edited by G. Nistico et al., Raven Press, New York 1986, pages 235-246). Groups of mice are pretreated by 30 min with the test compound by the oral or intraperitoneal routes and then given NMDA. Animals were observed for convulsions as defined by loss of righting reflex and appearance of tonic/clonic seizures. Animals are kept for 60 min after NMDA dosing and mortality was recorded.
b) NMDA receptor antagonist activity is measured in vitro by assaying a compounds ability to inhibit binding of the receptor antagonist 10,11-dihydro-5-methyl-5H-dibenzo[a,d]-cyclohepten-5,10-imine(MK801) to the receptor. The method is described by Foster and Wong, Br. J. Pharmacol. 91, 403-409 (1987).
c) NMDA and glycine receptor affinity may also be tested in the [³H]L-glutamate and [³H]glycine binding assays following the method of Monaghan & Cotman, PNAS, 83, 7532, (1986) and Watson et al, Neurosci. Res. Comm., 2, 169, (1988).

Certain compounds may act as neuromodulators by interfering with neurotransmitter uptake. Undesirable psychotomimetic effects may be associated with a compounds ability to inhibit dopamine uptake. Inhibition of dopamine uptake may be measured according to the method of Holtz et al, Molecular Pharmacol., 10, 746 (1974).

For the above-mentioned uses the dosage administered will, of course, vary with the compound employed, the mode of administration and the treatment desired. However, in general, satisfactory results are obtained when the compounds are administered at a daily dosage of from about 0.1 mg to about 20 mg per kg of animal body weight, preferably given in divided doses 1 to 4 times a day or in sustained release form. For man, the total daily dose is in the range of from 5 mg to 1,400 mg, more preferably from 10 mg to 100 mg, and unit dosage forms suitable for oral administration comprise from 2 mg to 1,400 mg of the compound admixed with a solid or liquid pharmaceutical carrier or diluent.

The compounds of formula I, and pharmaceutically acceptable derivatives thereof, may be used on their own or in the form of appropriate medicinal preparations for enteral or parenteral administration. According to the invention, there is also provided a pharmaceutical composition comprising preferably less than 80% and more preferably less than 50% by weight of a compound of formula I, or a pharmaceutically acceptable salt thereof, in admixture with a pharmaceutically acceptable adjuvant, diluent or carrier.

Examples of such adjuvants, diluents and carriers are: for tablets and dragees: lactose, starch, talc, stearic acid; for capsules: tartaric acid or lactose; for injectable solutions: water, alcohols, glycerin, vegetable oils; for suppositories: natural or hardened oils or waxes.

Compositions in a form suitable for oral, i.e. oesophageal administration include tablets, capsules and dragees;

Sustained release compositions include those in which the active ingredient is bound to an ion exchange resin which is optionally coated with a diffusion barrier to modify the release properties of the resin.

We prefer the composition to contain up to 50% and more preferably up to 25% by weight of the compound of formula I, or of the pharmaceutically acceptable derivative thereof.

The compounds of formula I and pharmaceutically acceptable derivatives thereof have the advantage that they are less toxic, more efficacious, are longer acting, have a broader range of activity, are more potent, produce fewer side effects, are more easily absorbed or have other useful pharmacological properties, compared to known compounds previously used in the therapeutic areas mentioned above.

The invention is illustrated, but in no way limited, by the following examples:

### Example 1

### N-Methyl-N-[1-(3-methyl-2-thienyl)-2-(2-aminopheny)ethyl]-2-aminoacetamide dihydrochloride

To an ice-cooled solution of N-(benzyloxycarbonyl)glycine(or CBZ-glycine) (9.31 g, 0.045 mol), N-hydroxysuccinimide (5.12 g, 0.045 mol), and a catalytic amount of 4-(dimethylamino)pyridine in dry THF (100 mL) was added dropwise a solution of dicyclohexylcarbodiimide in THF (75 mL). After 2 hours the reaction was filtered and the filtrate was added to N-methyl-α-(3-methyl-2-thienyl)-2-nitrobenzeneethanamine (4.1 g, 0.0148 mol). The reaction mixture was stirred overnight at room temperature. The reaction mixture was concentrated and the residue was suspended in ethyl acetate and the insolubles were filtered. Concentration of the ethyl acetate afforded the crude CBZ-glycine derivative which was purified by chromatography on silica gel and elution with 30% ethyl acetate-hexane to give a syrup (5 g). The syrup (5 g) was dissolved in glacial acetic acid (200 mL) and hydrogenated over 5% Pd/C at 40-50 psi for 16 hours. The catalyst was filtered off, a second charge of 5% Pd/C was added and the hydrogenation was continued for 24 hours. Filtration of the catalyst and concentration of the solvent afforded the crude diamine which was converted to the hydrochloride salt in isopropanol-ether to give the title product (1.2 g), mp. 175-178°C (decomp.)

### Example 2

### N-[1-(3-methyl-2-thienyl)-2-phenylethyl]-2-aminoacetamide hydrochloride.

To a stirred solution of 13.5 g (0.06 mol) of N-(benzyloxycarbonyl)glycine and 7.5 g (0.06 mol) of N-hydroxysuccinimide in 100 ml of dry tetrahydrofuran at 0°C was added dropwise a solution of 13.3 g (0.06 mol) of dicyclohexylcarbodiimide in 50 ml of tetrahydrofuran. The resulting mixture (white solid forms) was allowed to warm to ambient temperature and stirred overnight. To the filtrate obtained after filtering off the white solid was added, with stirring at ambient temperature, a solution of 14 g (.06 mol) of α-(3-methyl-2-thienyl)benzeneethanamine in 75 ml of tetrahydrofuran over a 30 minute period. The resulting mixture was stirred overnight and concentrated to near dryness to give an oil. Purification by silica gel chromatography gave 15 g of the N-CBZ intermediate as a pale yellow solid. A solution of the solid in 300 ml of glacial acetic acid with 16.5 g of 5% Pd/C catalyst was subjected up to 40 psi. of hydrogen in a Parr apparatus for 24 hours. The mixture was filtered through celite and the filtrate concentrated to near dryness using a toluene azeotrope. The resulting amber colored syrup was partitioned between chloroform (0.5L) and 4% aqueous sodium bicarbonate, the organic layer was washed with water and brine, and dried over sodium sulphate. Concentration of the organic solvents to dryness gave 8 g of crude amine as a syrup, which was dissolved in isopropanol, acidified with hydrogen chloride in isopropanol, and allowed to stand. The solid was collected and recrystallized from isopropanol to give 2.5 g of the hydrochloride as a white solid (mp 229-232°C).

### Example 3

By following essentially the same procedure as described for Example 2 but substituting;
N-methyl-α-(3-methyl-2-thienyl)benzeneethanamine;
N-ethyl-α-(2-thienyl)benzeneethanamine; or
N-ethyl-α-(3-methyl-2-thienyl)benzeneethanamine for the α-(3-methyl-2-thienyl)benzeneethanamine results in the preparation of;
N-methyl-N-[1-(3-methyl-2-thienyl)-2-phenylethyl]-2-aminoacetamide (Z)-2-butenedioate (1:2) salt (mp 128-130°C);
N-ethyl-N-[1-(2-thienyl)-2-phenylethyl]-2-aminoacetamide (Z)-2-butenedioate (1:1) salt (mp 134-138°C) ; or
N-ethyl-N-[1-(3-methyl-2-thienyl)-2-phenylethyl]-2-aminoacetamide (Z)-2-butenedioate (1:1.5) salt (mp 110-112°C).

### Example 4

### N-[1-(2-thienyl)-2-phenylethyl]-2-aminoacetamide maleate.

### a) N[1-(2-thienyl)-2-phenylethyl]-2-chloroacetamide.

A solution of chloroacetyl chloride (4 mL, 0.05 mol) in chloroform (40 mL) was added dropwise to a solution of α-(2-thienyl)benzeneethanamine (10g, 0.049 mol) and triethylamine (14 mL, 0.098 mol) in chloroform (180 mL) at 0°C. The mixture was stirred to room temperature for 1 hour. Dilute HCl (300 mL of 0.75N) was added and the reaction mixture was partitioned. The chloroform layer was separated, dried (MgSO₄) and concentrated to give the amide as a light brown solid (11 g).

### b) N-[1-(2-thienyl)-2-phenylethyl]-2-aminoacetamide maleate.

The amide (7 g, 0.025 mol) from step (a), dissolved in ethanol (100 mL) was charged to a bomb and cooled to -78°C. Liquid ammonia (25 mL) was added and the sealed bomb was heated at 75°-80°C overnight. The reaction was purified by chromatography on ammoniated silica gel and elution with 5%MeOH/CHCl₃ to give the product as an oil (3.5 g). The oil was dissolved in absolute ethanol (50 mL) and made acidic with maleic acid (1.9 g). The precipitated solid (2.8 g) was dried at 75°C for 4 hours to give the title compound, mp 165-166°C.

### Example 5

By following essentially the same procedure as described for Example 4 but substituting, α-methyl-α-(3-methyl-2-thienyl)benzeneethanamine, or α-methyl-α-(2-thienyl)benzeneethanamine for the α-(2-thienyl)benzeneethanamine results in the preparation of N-[1-methyl-1-(3-methyl-2-thienyl) -2-phenylethyl]-2-aminoacetamide fumarate, mp 199°-201°C, or N-[1-methyl-1-(2-thienyl)-2-phenylethyl]-2-aminoacetamide maleate, mp 174°-175°C.

### Example 6

### N-Ethyl-N-[1-(3-methyl-2-thienyl)-2-(2-aminophenyl)ethyl]-2-aminoacetamide maleate.

a) By following essentially the same procedures as described for Example 4, steps (a) and (b), but substituting N-ethyl-α-(3-methyl-2-thienyl)-2-nitrobenzeneethanamine for the α-(2-thienyl)benzeneethanamine results in the preparation of N-ethyl-N-[1-(3-methyl-2-thienyl)-2-(2-nitrophenyl)ethyl]-2-aminoacetamide.
b) The nitro compound (5 g) prepared according to the procedure of step (a) above was dissolved in absolute ethanol (100 mL) and hydrogenated over 10% Pd/C (3 g) in a Parr apparatus at 45-50 psi overnight. The catalyst was filtered and the filtrate concentrated to give an oily residue (3.7 g). The residue was dissolved in isopropanol and acidified with maleic acid. Ether was added to precipitate the salt which was isolated by filtration. The salt was dissolved in water and freeze-dried to give the title compound as a solid, mp 163°-168°C.

### Example 7

### N-[1-(3-Methyl-2-thienyl)-2-(2-aminophenyl)ethyl]-2-aminoacetamide dihydrochloride.

By following essentially the same procedures as described for Example 6 and substituting α-(3-methyl-2-thienyl)-2-nitrobenzeneethanamine for the N-ethyl-α-(3-methyl-2-thienyl)-2-nitrobenzeneethanamine results in the preparation of N-[1-(3-methyl-2-thienyl)-2-(2-aminophenyl)ethyl]-2-aminoacetamide. The base was converted to the hydrochloride salt with ethanolic hydrogen chloride and precipitated by the addition of ether to give the title compound as a solid, mp 206°-211°C.

### Example 8

The compound of Example 2 was tested for anticonvulsant activity against MES induced convulsions and found to have an ED₅₀(po) = 30.1 mg/kg.

## Claims (Claims for the following Contracting State(s): AT, BE, CH, LI, DE, DK, FR, GB, IT, LU, NL, SE)

1. A compound of formula I, 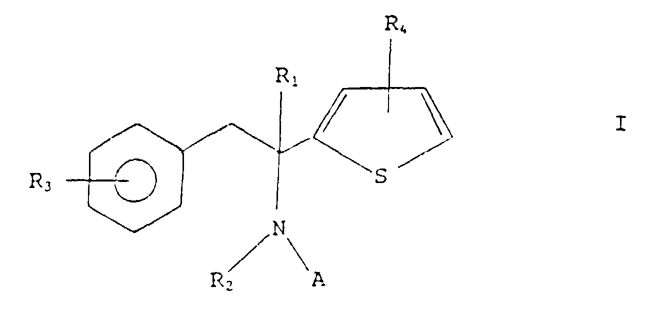 in which:
R₁ represents hydrogen or C₁₋₆ alkyl,
R₂ represents hydrogen, C₁₋₆ alkyl, C₃₋₆ alkenyl, C₃₋₆ alkynyl or C₃₋₆ cycloalkyl,
R₃ represents one or more radicals selected from hydrogen, hydroxy, C₁₋₆ alkyl, C₁₋₆ alkoxy, halogen, trifluoromethyl, amino, C₁₋₆ alkylamino or di(C₁₋₆ alkyl)amino,
R₄ represents one or more radicals selected from hydrogen or C₁₋₆ alkyl,
A represents OCCH₂NH₂,
and pharmaceutically acceptable acid addition salts thereof.

2. A compound according to claim 1 in which R₁ represents hydrogen or methyl.

3. A compound according to claim 1 or claim 2 in which R₄ represents hydrogen or methyl.

4. A compound according to any one of the preceding claims in which R₃ represents hydrogen or amino.

5. A compound according to claim 1 which is,
N-methyl-N-[1-(3-methyl-2-thienyl)-2-(2-aminophenyl)ethyl]-2-aminoacetamide,
N-[1-(3-methyl-2-thienyl)-2-phenylethyl]-2-aminoacetamide,
N-methyl-N-[1-(3-methyl-2-thienyl)-2-phenylethyl]-2-aminoacetamide,
N-ethyl-N-[1-(2-thienyl)-2-phenylethyl]aminoacetamide,
N-ethyl-N-[1-(3-methyl-2-thienyl)-2-phenylethyl]-2-aminoacetamide,
N-[1-(2-thienyl)-2-phenylethyl]-2-aminoacetamide,
N-[1-methyl-1-(3-methyl-2-thienyl)-2-phenylethyl]-2-aminoacetamide,
N-[1-methyl-1-(2-thienyl)-2-phenylethyl]-2-aminoacetamide
N-ethyl-N-[1-(3-methyl-2-thienyl)-2-(2-aminophenyl)ethyl]-2-aminoacetamide,
N-[1-(3-methyl-2-thienyl)-2-(2-aminophenyl)ethyl]-2-aminoacetamide,
or a pharmaceutically acceptable acid addition salt of any one thereof.

6. A pharmaceutical composition comprising a compound of formula I, as defined in any one of the preceding claims, or a pharmaceutically acceptable acid addition salt thereof, in association with a pharmaceutically acceptable adjuvant, diluent or carrier.

7. The use of a compound of formula I, as defined in any one of claims 1 to 5, and pharmaceutically acceptable acid addition salts thereof, in the manufacture of a medicament for use in the prevention and/or treatment of neurological disorders.

8. A process for the preparation of compounds of formula I, as defined in any one of claims 1 to 5, or pharmaceutically acceptable acid addition salts thereof, which comprises
(a) producing a compound of formula I by reacting the corresponding compound of formula I in which A represents hydrogen with a compound of the formula II, 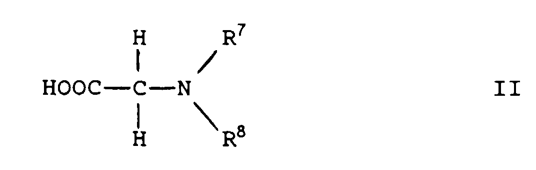 or a carboxyl activated derivative thereof, in which both R₇ and R₈ represent a nitrogen protecting group or one of R₇ and R₈ represents a nitrogen protecting group and the other represents hydrogen; or
(b) producing a compound of formula I by reacting the corresponding compound of formula I in which A represents COCH₂X and X represents a suitable leaving group with ammonia; or
(c) producing a compound of the formula I in which one of R₃ represents an NH₂ group by reduction of the corresponding compound of formula I in which one of R₃ represents a NO₂ group; or
(d) producing a compound of formula I containing one or more amino or hydroxy groups by removing a protecting group from a compound of formula I in which one or more of the amino or hydroxyl groups is protected;
and where desired or necessary converting the resulting compound of formula I into a pharmaceutically acceptable acid addition salt thereof or vice versa.

## Claims (Claims for the following Contracting State(s): ES, GR)

1. A process for preparation of a compound of formula I, 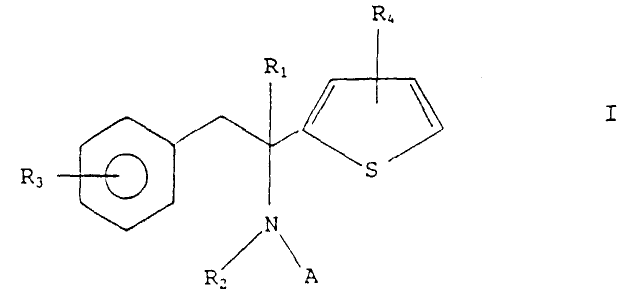 in which:
R₁ represents hydrogen or C₁₋₆ alkyl,
R₂ represents hydrogen, C₁₋₆ alkyl, C₃₋₆ alkenyl, C₃₋₆ alkynyl or C₃₋₆ cycloalkyl,
R₃ represents one or more radicals selected from hydrogen, hydroxy, C₁₋₆ alkyl, C₁₋₆ alkoxy, halogen, trifluoromethyl, amino, C₁₋₆ alkylamino or di(C₁₋₆ alkyl)amino,
R₄ represents one or more radicals selected from hydrogen or C₁₋₆ alkyl,
A represents OCCH₂NH₂,
and pharmaceutically acceptable acid addition salts thereof, which comprises:
(a) producing a compound of formula I by reacting the corresponding compound of formula I in which A represents hydrogen with a compound of the formula II, 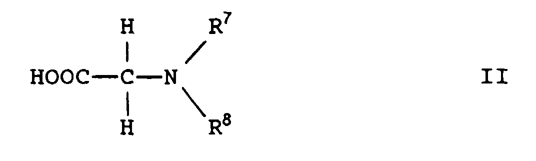 or a carboxyl activated derivative thereof, in which both R₇ and R₈ represent a nitrogen protecting group or one of R₇ and R₈ represents a nitrogen protecting group and the other represents hydrogen; or
(b) producing a compound of formula I by reacting the corresponding compound of formula I in which A represents COCH₂X and X represents a suitable leaving group with ammonia; or (c) producing a compound of the formula I in which one of R₃ represents an NH₂ group by reduction of the corresponding compound of formula I in which one of R₃ represents a NO₂ group; or
(d) producing a compound of formula I containing one or more amino or hydroxy groups by removing a protecting group from a compound of formula I in which one or more of the amino or hydroxyl groups is protected;
and where desired or necessary converting the resulting compound of formula I into a pharmaceutically acceptable acid addition salt thereof or vice versa.

2. A process according to claim 1 in which R₁ represents hydrogen or methyl.

3. A process according to claim 1 or claim 2 in which R₄ represents hydrogen or methyl.

4. A process according to any one of the preceding claims in which R₃ represents hydrogen or amino.

5. A process according to claim 1 in which the compound of formula I is:
N-methyl-N-[1-(3-methyl-2-thienyl)-2-(2-aminophenyl)ethyl]-2-aminoacetamide,
N-[1-(3-methyl-2-thienyl)-2-phenylethyl]-2-aminoacetamide,
N-methyl-N-[1-(3-methyl-2-thienyl)-2-phenylethyl]-2-aminoacetamide,
N-ethyl-N-[1-(2-thienyl)-2-phenylethyl]-2-aminoacetamide,
N-ethyl-N-[1-(3-methyl-2-thienyl)-2-phenylethyl]-2-aminoacetamide,
N-[1-(2-thienyl)-2-phenylethyl]-2-aminoacetamide,
N-[1-methyl-1-(3-methyl-2-thienyl)-2-phenylethyl]-2-aminoacetamide,
N-[1-methyl-1-(2-thienyl)-2-phenylethyl]-2-aminoacetamide
N-ethyl-N-[1-(3-methyl-2-thienyl)-2-(2-aminophenyl)ethyl]-2-aminoacetamide,
N-[1-(3-methyl-2-thienyl)-2-(2-aminophenyl)ethyl]-2-aminoacetamide,
or a pharmaceutically acceptable acid addition salt of any one thereof.

## Patentansprüche (Patentansprüche für folgende(n) Vertragsstaat(en): AT, BE, CH, LI, DE, DK, FR, GB, IT, LU, NL, SE)

1. Verbindung der Formel I 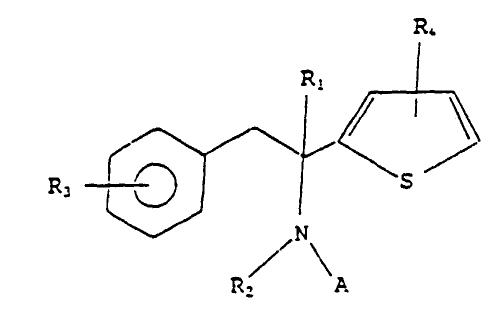 worin:
R₁ für Wasserstoff oder C₁₋₆-Alkyl,
R₂ für Wasserstoff, C₁₋₆-Alkyl, C₃₋₆-Alkenyl, C₃₋₆-Alkinyl oder C₃₋₆-Cycloalkyl,
R₃ für einen oder mehrere unter Wasserstoff, Hydroxyl, C₁₋₆-Alkyl, C₁₋₆-Alkoxy, Halogen, Trifluormethyl, Amino, C₁₋₆-Alkylamino oder Di(C₁₋₆-Alkyl)amino ausgewählte Reste,
R₄ für einen oder mehrere unter Wasserstoff und C₁₋₆-Alkyl ausgewählte Reste und
A für OCCH₂N₂ steht,
und deren pharmazeutisch annehmbare Säureadditionssalze.

2. Verbindung nach Anspruch 1, worin R₁ für Wasserstoff oder Methyl steht.

3. Verbindung nach Anspruch 1 oder 2, worin R₄ für Wasserstoff oder Methyl steht.

4. Verbindung nach einem der vorhergehenden Ansprüche, worin R₃ für Wasserstoff oder Amino steht.

5. Verbindung nach Anspruch 1, bei der es sich um
N-Methyl-N-[1-(3-methyl-2-thienyl)-2-(2-aminophenyl)ethyl]-2-aminoacetamid,
N-[1-(3-Methyl-2-thienyl)-2-phenylethyl]
N-Methyl-N-[1-(3-methyl-2-thienyl)-2-phenylethyl]-2-aminoacetamid,
N-Ethyl-N-[1-(2-thienyl)-2-phenylethyl]-2-aminoacetamid,
N-Ethyl-N-[1-(3-methyl-2-thienyl)-2-phenylethyl]-2-aminoacetamid,
N-[1-(2-Thienyl)-2-phenylethyl]-2-aminoacetamid,
N-[1-Methyl-1-(3-methyl-2-thienyl)-2-phenylethyl]-2-aminoacetamid,
N-[1-Methyl-1-(2-thienyl)-2-phenylethyl]-2-aminoacetamid,
N-Ethyl-N-[1-(3-methyl-2-thienyl)-2-(2-aminophenyl)ethyl]-2-aminoacetamid,
N-[1-(3-Methyl-2-thienyl)-2-(2-aminophenyl)ethyl]-2-aminoacetamid
oder ein pharmazeutisch annehmbares Säureadditionssalz davon handelt.

6. Pharmazeutische Zusammensetzung, enthaltend eine Verbindung der Formel I gemäß einem der vorhergehenden Ansprüche oder ein pharmazeutisch annehmbares Säureadditionssalz davon, zusammen mit einem pharmazeutisch annehmbaren Hilfsstoff, Verdünnungsmittel oder Träger.

7. Verwendung einer Verbindung der Formel I gemäß einem der Ansprüche 1 bis 5 und deren pharmazeutisch annehmbaren Säureadditionssalzen bei der Herstellung eines Arzneimittels zur Verwendung bei der Prävention und/oder der Behandlung von neurologischen Erkrankungen.

8. Verfahren zur Herstellung von Verbindungen der Formel I gemäß einem der Ansprüche 1 bis 5 oder deren pharmazeutisch annehmbaren Säureadditionssalzen, bei dem man
(a) eine Verbindung der Formel I herstellt, indem man die entsprechende Verbindung der Formel I, worin A für Wasserstoff steht, mit einer Verbindung der Formel II 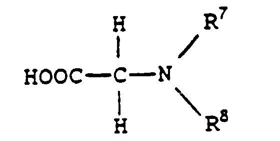 oder einem carboxy-aktivierten Derivat davon, worin sowohl R₇ als auch R₈ jeweils für eine Stickstoff-Schutzgruppe stehen oder entweder R₇ oder R₈ für eine Stickstoff-Schutzgruppe und die jeweils andere Gruppe für Wasserstoff steht, umsetzt; oder
(b) eine Verbindung der Formel I herstellt, indem man die entsprechende Verbindung der Formel I, worin A für COCH₂X steht und X eine geeignete Abgangsgruppe bedeutet, mit Ammoniak umsetzt; oder
(c) eine Verbindung der Formel I, worin eine der Gruppen R₃ für eine N₂-Gruppe steht, herstellt, indem man die entsprechende Verbindung der Formel I, worin eine der Gruppen R₃ eine NO₂-Gruppe bedeutet, reduziert; oder
(d) eine eine oder mehrere Amino- oder Hydroxylgruppen enthaltende Verbindung der Formel I herstellt, indem man von einer Verbindung der Formel I, worin eine oder mehrere der Amino- oder Hydroxylgruppen geschützt sind, eine Schutzgruppe abspaltet,
und wobei man gegebenenfalls die erhaltene Verbindung der Formel I in ein pharmazeutisch annehmbares Säureadditionssalz davon überführt oder umgekehrt.

## Patentansprüche (Patentansprüche für folgende(n) Vertragsstaat(en): ES, GR)

1. Verfahren zur Herstellung einer Verbindung der Formel I 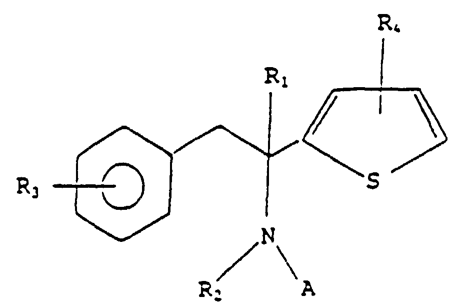 worin:
R₁ für Wasserstoff oder C₁₋₆-Alkyl,
R₂ für Wasserstoff, C₁₋₆-Alkyl, C₃₋₆-Alkenyl, C₃₋₆-Alkinyl oder C₃₋₆-Cycloalkyl,
R₃ für einen oder mehrere unter Wasserstoff, Hydroxyl, C₁₋₆-Alkyl, C₁₋₆-Alkoxy, Halogen, Trifluormethyl, Amino, C₁₋₆-Alkylamino oder Di(C₁₋₆-Alkyl)amino ausgewählte Reste,
R₄ für einen oder mehreren unter Wasserstoff und C₁₋₆-Alkyl ausgewählte Reste und
A für OCCH₂NH₂ steht,
und deren pharmazeutisch annehmbaren Säureadditionssalzen, bei dem man
(a) eine Verbindung der Formel I herstellt, indem man die entsprechende Verbindung der Formel I, worin A für Wasserstoff steht, mit einer Verbindung der Formel II 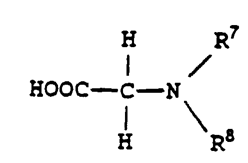 oder einem carboxy-aktivierten Derivat davon, worin sowohl R₇ als auch R₈ jeweils für eine Stickstoff-Schutzgruppe stehen oder entweder R₇ oder R₈ für eine Stickstoff-Schutzgruppe und die jeweils andere Gruppe für Wasserstoff steht, umsetzt; oder
(b) eine Verbindung der Formel I herstellt, indem man die entsprechende Verbindung der Formel I, worin A für COCH₂X steht und X eine geeignete Abgangsgruppe bedeutet, mit Ammoniak umsetzt; oder
(c) eine Verbindung der Formel I, worin eine der Gruppen R₃ für eine N₂-Gruppe steht, herstellt, indem man die entsprechende Verbindung der Formel I, worin eine der Gruppen R₃ eine NO₂-Gruppe bedeutet, reduziert; oder
(d) eine eine oder mehrere Amino- oder Hydroxylgruppen enthaltende Verbindung der Formel I herstellt, indem man von einer Verbindung der Formel I, worin eine oder mehrere der Amino- oder Hydroxylgruppen geschützt sind, eine Schutzgruppe abspaltet,
und wobei man gegebenenfalls die erhaltene Verbindung der Formel I in ein pharmazeutisch annehmbares Säureadditionssalz davon überführt oder umgekehrt.

2. Verfahren nach Anspruch 1, worin R₁ für Wasserstoff oder Methyl steht.

3. Verfahren nach Anspruch 1 oder 2, worin R₄ für Wasserstoff oder Methyl steht.

4. Verfahren nach einem der vorhergehenden Ansprüche, worin R₃ für Wasserstoff oder Amino steht.

5. Verfahren nach Anspruch 1, wobei es sich bei der Verbindung der Formel I um
N-Methyl-N-[1-(3-methyl-2-thienyl)-2-(2-aminophenyl)ethyl]-2-aminoacetamid,
N-[1-(3-Methyl-2-thienyl)-2-phenylethyl]-2-aminoacetamid,
N-Methyl-N-[1-(3-methyl-2-thienyl)-2-phenylethyl]-2-aminoacetamid,
N-Ethyl-N-[1-(2-thienyl)-2-phenylethyl]-2-aminoacetamid,
N-Ethyl-N-[1-(3-methyl-2-thienyl)-2-phenylethyl]-2-aminoacetamid,
N-[1-(2-Thienyl)-2-phenylethyl]-2-aminoacetamid,
N-[1-Methyl-1-(3-methyl-2-thienyl)-2-phenylethyl]-2-aminoacetamid,
N-[1-Methyl-1-(2-thienyl)-2-phenylethyl]-2-aminoacetamid,
N-Ethyl-N-[1-(3-methyl-2-thienyl)-2-(2-aminophenyl)ethyl]-2-aminoacetamid,
N-[1-(3-Methyl-2-thienyl)-2-(2-aminophenyl)ethyl]-2-aminoacetamid
oder ein pharmazeutisch annehmbares Säureadditionssalz davon handelt.

## Revendications (Revendications pour l'(les) Etat(s) contractant(s) suivant(s): AT, BE, CH, LI, DE, DK, FR, GB, IT, LU, NL, SE)

1. Composé de formule I, 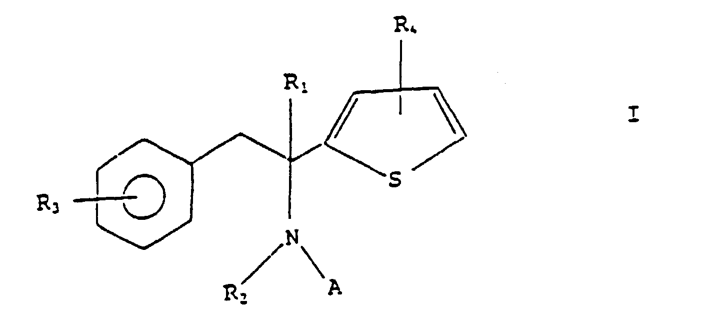 dans laquelle:
R₁ représente l'hydrogène ou alkyle en C₁₋₆,
R₂ représente l'hydrogène, alkyle en C₁₋₆, alkényle en C₃₋₆, alkynyle en C₃₋₆ ou cycloalkyle en C₃₋₆,
R₃ représente un ou plusieurs radicaux choisis parmi l'hydrogène, hydroxy, alkyle en C₁₋₆, alcoxy en C₁₋₆, halogène, trifluorométhyle, amino, alkylamino en C₁₋₆ ou dialkyle(C₁₋₆)-amino,
R₄ représente un ou plusieurs radicaux choisis parmi l'hydrogène ou alkyle en C₁₋₆,
A représente OCCH₂NH₂,
et les sels d'addition d'acide pharmaceutiquement acceptables de celui-ci.

2. Composé selon la revendication 1, caractérisé en ce que R₁ représente l'hydrogène ou méthyle.

3. Composé selon la revendication 1 ou la revendication 2, caractérisé en ce que R₄ représente l'hydrogène ou méthyle.

4. Composé selon l'une quelconque des revendications précédentes, caractérisé en ce que R₃ représente l'hydrogène ou amino.

5. Composé selon la revendication 1, caractérisé en ce qu'il s'agit de,
N-méthyl-N-[1-(3-méthyl-2-thiényl)-2-(2-aminophényl)éthyl]-2-aminoacétamide,
N-[1-(3-méthyl-2-thiényl)-2-phényléthyl]-2-aminoacétamide,
N-méthyl-N-[1-(3-méthyl-2-thiényl)-2-phényléthyl]-2-aminoacétamide,
N-éthyl-N-[1-(2-thiényl)-2-phényléthyl]-2-aminoacétamide,
N-éthyl-N-[1-(3-méthyl-2-thiényl)-2-phényléthyl]-2-aminoacétamide,
N-[1-(2-thiényl)-2-phényléthyl]-2-aminoacétamide,
N-[1-méthyl-1-(3-méthyl-2-thiényl)-2-phényléthyl]-2-aminoacétamide,
N-[1-méthyl-1-(2-thiényl)-2-phényléthyl]-2-aminoacétamide,
N-éthyl-N-[1-(3-méthyl-2-thiényl)-2-(2-aminophényl)éthyl]-2-aminoacétamide,
N-[1-(3-méthyl-2-thiényl)-2-(2-aminophényl)éthyl]-2-aminoacétamide,
ou un sel d'addition d'acide pharmaceutiquement acceptable de l'un quelconque d'entre eux.

6. Composition pharmaceutique comprenant un composé de formule I, tel que défini dans l'une quelconque des revendications précédentes, ou un sel d'addition d'acide pharmaceutiquement acceptable de celui-ci, en association avec un adjuvant, un diluant ou un support pharmaceutiquement acceptable.

7. Utilisation d'un composé de formule I, tel que défini dans l'une quelconque des revendications 1 à 5, et les sels d'addition d'acide pharmaceutiquement acceptables de celui-ci, dans la fabrication d'un médicament destiné à être utilisé dans la prévention et/ou le traitement de troubles neurologiques.

8. Procédé de préparation de composés de formule I, tel que définis dans l'une quelconque des revendications 1 à 5, ou les sels d'addition d'acide pharmaceutiquement acceptable de ceux-ci, comprenant
(a) la production d'un composé de formule I par réaction du composé correspondant de formule I, dans laquelle A représente l'hydrogène, avec un composé de formule II, 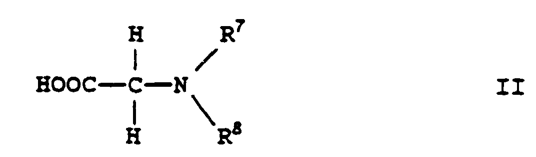 ou un dérivé carboxy activé de celui-ci, dans laquelle à la fois R₇ et R₈ représentent un groupement protecteur d'azote ou l'un parmi R₇ et R₈ représente parmi un groupement protecteur d'azote et l'autre représente l'hydrogène; ou
(b) la production d'un composé de formule I par réaction du composé correspondant de formule I dans laquelle A représente COCH₂X et X représente un groupement partant approprié, avec l'ammoniac; ou
(c) la production d'un composé de formule I dans laquelle l'un des R₃ représente un groupement NH₂, par réduction du composé correspondant de formule I dans laquelle l'un des R₃ représente un groupement NO₂; ou
(d) la production d'un composé de formule I, contenant un ou plusieurs groupements amino ou hydroxy, par l'élimination d'un groupement protecteur d'un composé de formule I, dans laquelle un ou plusieurs des groupements amino ou hydroxy sont protégés;
et si on le souhaite ou si nécessaire, la transformation du composé résultant de formule I en un sel d'addition d'acide pharmaceutiquement acceptable de celui-ci ou vice versa.

## Revendications (Revendications pour l'(les) Etat(s) contractant(s) suivant(s): ES, GR)

1. Procédé de préparation d'un composé de formule I, 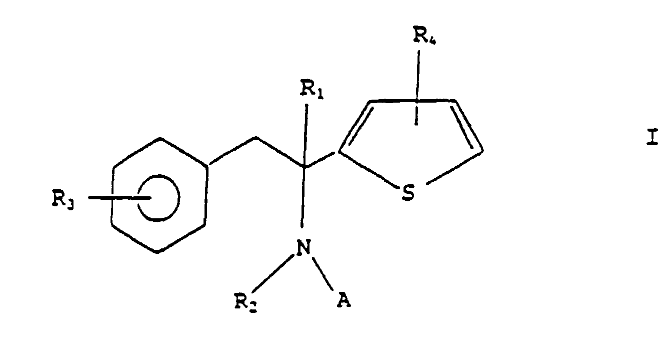 dans laquelle:
R₁ représente l'hydrogène ou alkyle en C₁₋₆,
R₂ représente l'hydrogène, alkyle en C₁₋₆, alkényle en C₃₋₆, alkynyle en C₃₋₆ ou cycloalkyle en C₃₋₆,
R₃ représente un ou plusieurs radicaux choisis parmi l'hydrogène, hydroxy, alkyle en C₁₋₆, alcoxy en C₁₋₆, halogène, trifluorométhyle, amino, alkylamino en C₁₋₆ ou dialkyle(C₁₋₆)-amino,
R₄ représente un ou plusieurs radicaux choisis parmi l'hydrogène ou alkyle en C₁₋₆,
A représente OCCH₂NH₂,
et les sels d'addition d'acide pharmaceutiquement acceptable de celui-ci, comprenant:
(a) la production d'un composé de formule I par réaction du composé correspondant de formule I, dans laquelle A représente l'hydrogène, avec un composé de formule II, 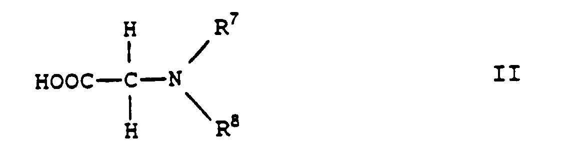 ou un dérivé carboxy activé de celui-ci, dans laquelle à la fois R₇ et R₈ représentent un groupement protecteur d'azote ou l'un de R₇ et R₈ représentent un groupement protecteur d'azote et l'autre représente l'hydrogène; ou
(b) la production d'un composé de formule I par réaction du composé correspondant de formule I dans laquelle A représente COCH₂X et X représente un groupement partant approprié, avec l'ammoniac; ou
(c) la production d'un composé de formule I dans laquelle l'un des R₃ représente un groupement NH₂, par réduction du composé correspondant de formule I dans laquelle l'un des R₃ représente un groupement NO₂; ou
(d) la production d'un composé de formule I, contenant un ou plusieurs groupements amino ou hydroxy, par l'élimination d'un groupement protecteur d'un composé de formule I, dans laquelle un ou plusieurs des groupements amino ou hydroxy sont protégés;
et si on le souhaite ou si nécessaire, la transformation du composé résultant de formule I en un sel d'addition d'acide pharmaceutiquement acceptable de celui-ci ou vice versa.

2. Procédé selon la revendication 1, caractérisé en ce que R₁ représente l'hydrogène ou méthyle.

3. Procédé selon la revendication 1 ou la revendication 2, caractérisé en ce que R₄ représente l'hydrogène ou méthyle.

4. Procédé selon l'une quelconque des revendications précédentes, caractérisé en ce que R₃ représente l'hydrogène ou amino.

5. Procédé selon la revendication 1, caractérisé en ce que le composé de formule I est,
le N-méthyl-N-[1-(3-méthyl-2-thiényl)-2-(2-aminophényl)éthyl]-2-aminoacétamide,
le N-[1-(3-méthyl-2-thiényl)-2-phényléthyl]-2-aminoacétamide,
le N-méthyl-N-[1-(3-méthyl-2-thiényl)-2-phényléthyl]-2-aminoacétamide,
le N-éthyl-N-[1-(2-thiényl)-2-phényléthyl]-2-aminoacétamide,
le N-éthyl-N-[1-(3-méthyl-2-thiényl)-2-phényléthyl]-2-aminoacétamide,
le N-[1-(2-thiényl)-2-phényléthyl]-2-aminoacétamide,
le N-[1-méthyl-1-(3-méthyl-2-thiényl)-2-phényléthyl]-2-aminoacétamide,
le N-[1-méthyl-1-(2-thiényl)-2-phényléthyl]-2-aminoacétamide,
le N-éthyl-N-[1-(3-méthyl-2-thiényl)-2-(2-aminophényl)éthyl]-2-aminoacétamide,
le N-[1-(3-méthyl-2-thiényl)-2-(2-aminophényl)éthyl]-2-aminoacétamide,
ou un sel d'addition d'acide pharmaceutiquement acceptable de l'un quelconque d'entre eux.
